# EUROPEAN PATENT SPECIFICATION

(11) **EP 2 455 028 B1**
(45) Date of publication and mention of the grant of the patent: **17.02.2016**
(21) Application number: 10192079.1
(22) Date of filing: 22.11.2010
(51) Int. Cl.: A61B 17/70

(54) **Polyaxial bone anchoring device**
Polyaxiale Knochenverankerungsvorrichtung
Dispositif d'ancrage d'os polyaxial

(43) Date of publication of application: 23.05.2012
(73) Proprietor: Biedermann Technologies GmbH & Co. KG, 78166 Donaueschingen (DE)
(72) Inventor: Biedermann, Lutz, 78048, VS-Villingen (DE); Pohl, Gerhard, 78112, St. Georgen (DE)
(74) Representative: Prüfer & Partner mbB Patentanwälte · Rechtsanwälte

(56) References cited:
- WO-A1-2010/103198
- US-A- 5 672 176
- US-A- 5 681 319
- US-A1- 2006 264 933
- US-A1- 2009 062 867
- US-A1- 2010 137 920
- US-A1- 2010 160 977

## Description

The invention relates to a polyaxial bone anchoring device for anchoring a stabilization rod in a bone or in a vertebra. The bone anchoring device includes an anchoring element, a receiving part for receiving a head of the bone anchoring element and for receiving a stabilization rod to be connected to the anchoring element. The anchoring element is pivotably connected to the receiving part and can be locked at an angle by exerting pressure onto the head via a pressure element that is arranged in the receiving part. The pressure element and the receiving part are configured to cooperate in such a way that the pressure element frictionally clamps the head to maintain a desired angular position before locking.

US 5,716,356 describes a polyaxial bone screw including a screw element and a receiving part which is pivotably connected to the screw element and a pressure element to exert pressure onto the head of the screw element to lock the angle between the screw element and the receiving part. The receiving part has a U-shaped channel for receiving a stabilization rod. The pressure element comprises a cylindrical recess, which is to be aligned with the U-shaped channel to receive the rod therein. In order to hold the pressure element in a position aligned with the U-shaped channel, the position of the pressure element is fixed by crimping through bores provided in the receiving part.

When the head of the bone anchoring element is freely pivotable with respect to the receiving part before locking the head in a final angular position, the alignment of the receiving part and the insertion of the rod may be difficult in more complex clinical applications, for example, when a multitude of bone anchors have to be connected to the rod.

US 7,604,656 describes a fastener engageable with a bone portion to connect a longitudinal member to the bone portion. The housing that receives the fastener also receives a spacer, which is engageable with the fastener and the longitudinal member. In one embodiment, the spacer is urged by a pin member into frictional engagement with the fastener and with the housing.

US 2004/0267264 A1 describes a polyaxial fixation device, wherein the polyaxial bone screw includes an engagement member that is adapted to provide sufficient friction between the spherical head and the receiver member to enable the shank to be maintained in a desired angular orientation before locking the spherical head within the receiver member. The engagement member is realized, for example, by a snap ring around the head or by spring members provided at the compression cap to frictionally engage the spherical head or by a slot provided in the compression cap.

US 5,681,319 A discloses a bone anchoring element comprising a bone anchor having a threaded shaft for anchoring in a bone and a head, a receiving part, a pressure element and two locking devices.

US 5,672,176 A discloses an anchoring member for connecting a rod with a bone, said anchoring member comprising a screw member, a seat part receiving the screw head and said rod, the seat part has a first portion having a substantially U-shaped cross-section with two free legs with an internal screw thread for receiving said rod therebetween, and a second portion, said second portion tapering with a predetermined cone angle, and a pressure member formed to embrace said screw head from its side opposite to said threaded portion and having an outer conical surface in a region laterally surrounding said screw head, said conical surface tapering towards said second end with a cone angle corresponding to said predetermined cone angle.

It is an object of the invention to provide a polyaxial bone anchoring device, which allows an improved handling during surgery and which can be manufactured in a simple manner.

The object is solved by a polyaxial bone anchoring device according to claim 1 or 13 and by a method for manufacturing the polyaxial bone anchoring device according to claim 15. Further developments are given in the dependent claims.

With the polyaxial bone anchoring device a temporary clamping of the head in a desired angular position with respect to the receiving part without locking the head can be achieved. This allows to maintain the receiving part in an adjustable angular position. In this condition, the pressure element exerts a preload onto the head in which the head is not locked but prevented from freely pivoting. When the head is temporarily clamped, the alignment of the receiving part with respect to the rod and the insertion of the rod is facilitated, in particular in a situation in which a multitude of bone anchors have to be connected to the rod.

When the rod is already inserted into the receiving part, adjustments of the rod are still possible without completely loosening the head.

The polyaxial bone anchoring device comprises only few parts which are of simple design. The mechanism to frictionally maintain the head before locking it is free from any spring members or portions. This facilitates the manufacturing of the polyaxial bone anchoring device. Further-more, existing receiving parts and pressure elements can be used without having to redesign their shape. It is possible to simply change the location of the crimp bores.

The amount of preload exerted onto the head by the pressure member can be exactly predefined in a simple manner by selecting the position and shape of the crimp bores. The polyaxial bone anchoring device is provided to the surgeon in a pre-assembled manner, in which the pressure element is axially and rotationally fixed to such an extent that it can not fall out or be rotated out of its aligned position. This allows a safe handling by the surgeon.

The receiving part and the pressure element can be manufactured in series at low costs.

According to an aspect, a method for manufacturing (which is not claimed) a polyaxial bone anchoring device according to the invention as claimed includes the steps
arranging the pressure element in the receiving part such that the recess is located with respect to the deformable portion in an axial direction in such a way that the deformable portion can engage upon deformation an edge of the recess;
deforming the deformable portion such that the deformed portion forms a protrusions, which engages the edge of the recess, thereby exerting a force onto the pressure element to maintain the head in an angular position by friction before locking it.

The recess may formed at the pressure element and the protrusion may be formed at the receiving part and wherein the protrusion is located above a lower edge of the recess.

The recess may also be formed at the receiving part and the protrusion may be formed at the pressure element and wherein the protrusion is located below an upper edge of the recess.

The deformation may be made by crimping

Further features and advantages of the invention will become apparent from the description of embodiments by means of the accompanying drawings.

In the drawings:
- Fig. 1: shows a perspective exploded view of the polyaxial bone anchoring device according to a first embodiment.
- Fig. 2: shows the polyaxial bone anchoring device of Fig. 1 in an assembled state.
- Fig. 3: shows a cross-sectional view of the polyaxial bone anchoring device in an assembled state before final locking of the head.
- Fig. 4a: shows a cross-sectional view of the polyaxial bone anchoring device before provisionally fixing the pressure element in the receiving part.
- Fig. 4b: shows an enlarged portion of Fig. 4a.
- Fig. 5a: shows a cross-sectional view of the polyaxial bone anchoring device in the pre-assembled state after provisionally fixing of the pressure element in the receiving part.
- Fig. 5b: shows an enlarged portion of Fig. 5a.
- Fig. 6: shows a cross-sectional view of a tool for provisionally fixing the pressure element in the receiving part.
- Fig. 7: shows an enlarged portion of Fig. 6.
- Fig. 8: shows a cross-sectional view of a modified embodiment of the polyaxial bone anchoring device before locking of the head.
- Fig. 9: shows a cross-sectional view of a further modified embodiment of the polyaxial bone anchoring device in a state before locking of the head.
- Fig. 10: shows a perspective exploded view of a second embodiment of the polyaxial bone anchoring device.
- Fig. 11: shows an enlarged cross-sectional view of a portion of the pressure element of the bone anchoring device of Fig. 10.
- Fig. 12: shows a cross-sectional view of the polyaxial bone anchoring device of the second embodiment before provisionally fixing the pressure element in the receiving part.
- Fig. 13: shows a cross-sectional view of the polyaxial bone anchoring device of Fig. 12 in the pre-assembled state after provisionally fixing of the pressure element in the receiving part.

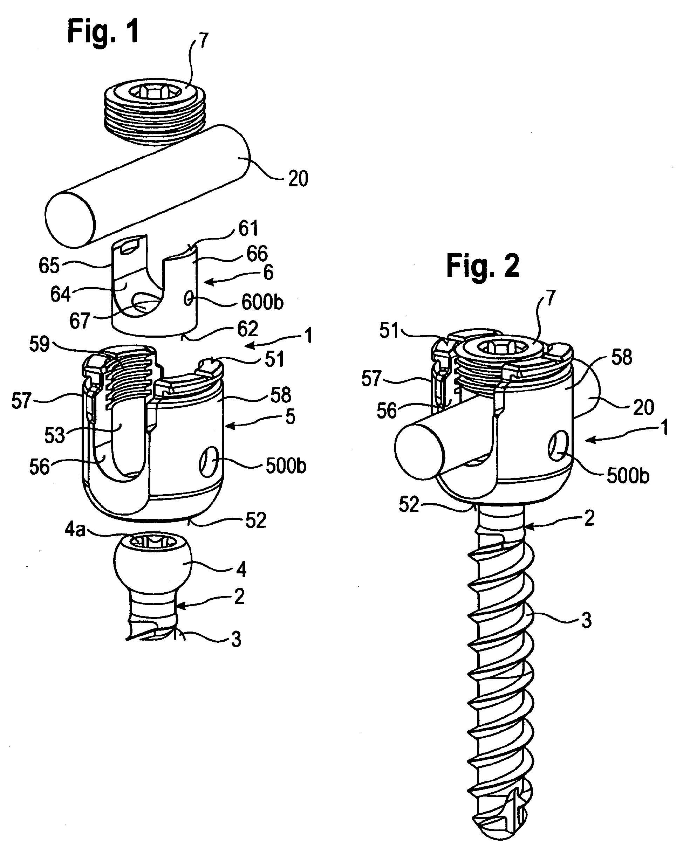

The polyaxial bone anchoring device 1 according to a first embodiment as shown in Figs. 1 to 3 includes a bone anchoring element in the form of a screw member 2 having a threaded shaft 3 and a head 4. The head 4 is generally spherical and includes a recess 4a at its free end for engagement with a tool to insert the threaded shaft 3 into bone. The bone anchoring device further includes a receiving part 5 for connecting the screw member 2 to a rod 20. A pressure element 6 is arranged in the receiving part on top of the head 4. For securing the rod 20 in the receiving part and for exerting pressure onto the head, a locking device, for example an inner screw 7, which cooperates with the receiving part 5, is provided.

The receiving part is a substantially cylindrical one piece part and has a top end 51 and a bottom end 52. A passageway extending from the top end to the bottom end is formed by a coaxial bore 53 followed by a seat portion 54 for receiving the head 4 of the screw member 2. The seat portion 54 has an opening 55 at the bottom end 52 through which the shaft 3 of the screw member extends. The seat portion 54 is shown to be spherically-shaped, but it can be tapered or it can have any other shape that allows to receive the head 4 so that it can pivot with respect to the receiving part 5. At the top end 51 a substantially U-shaped recess 56 is provided by means of which two free legs 57, 58 are formed that are the sidewalls of a channel for receiving the rod 20. An internal thread 59 is provided at the legs for cooperating with the inner screw 7.

The pressure element 6 is formed in one piece. It is of substantially cylindrical construction and has an outer diameter, which allows it to move in the axial direction within the bore 53 of the receiving part 5. The pressure element 6 has a top end 61 and a bottom end 62. When the pressure element is inserted into the receiving part, the bottom end 62 faces the head 4 of the screw element 2. At the bottom end 62 a spherical recess 63 is provided, which is adapted to the size and shape of the head 4. The spherical recess is configured to come into frictional engagement with the spherical surface of the head. At the top end 61, a U-shaped recess 64 is provided by means of which two free legs 65, 66 are formed that form a channel to receive the rod 20 therein. Furthermore, the pressure element 6 includes a coaxial bore 67 for accessing the screw head 4 with a tool (not shown). As shown in the Figs., the pressure element 6 is a solid member without any spring portions, which could render it flexible. It is arranged in the receiving part such that the U-shaped recess 56 of the receiving part 5 and the U-shaped recess 64 of the pressure element are aligned.

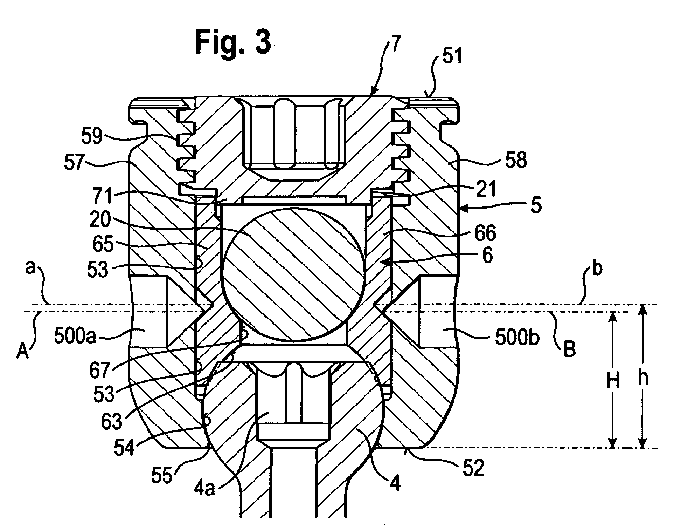

In the assembled state as shown in Fig. 3, the screw head 4 is located in the seat 54 and the pressure element 6 is arranged on top of the screw head 4. The height of the free legs 65, 66 of the pressure element is configured such that the free legs 65, 66 extend above the rod 20 when the rod is inserted and rests on the bottom of the channel.

The locking device in the form of the inner screw 7 has a projection 71 extending into the channel formed by the free legs 65, 66 of the pressure element 6. The size of the projection 71 in an axial direction is such that when the inner screw 7 is tightened, the projection 71 presses onto the rod while there is still a gap 21 between the top end 61 of the pressure element and the lower side of the inner screw 7. Therefore, with the single inner screw 7 pressure can be exerted onto the rod 20 only, which in turn can exert pressure onto the pressure element 6. It should be noted that instead of the single part locking device in form of the inner screw 7 a two-part locking device can be used (not shown). The two-part locking device includes a first part to be screwed in-between the legs 57, 58 of the receiving part. The first part acts onto the top end 61 of the pressure element 6. Further, a second part in form of an inner screw is provided in the first part, which presses onto the rod 20. By means of this, the head 4 and the rod 5 can be independently fixed.

The pressure element 6 is retained in the receiving part 5 as shown in Figs. 3 to 5. As shown in particular in 4a and 4b, the receiving part includes two blind holes 500a, 500b forming crimp bores that extend from the outer surface to a distance from the inner wall of the coaxial bore 53. The blind holes 500a, 500b are arranged at 180° offset from each other and at 90° with respect to the channel formed by the U-shaped recess 56. The blind holes 500a, 500b are aligned perpendicular with respect to the bore axis M of the coaxial bore 53. At their end they are tapered with an angle α preferably less than 45°, for example 22,5°, with respect to an axis parallel to the bore axis M. The bore axes A and B of the blind holes 500a, 500b are provided at a distance H from the second end 52 of the receiving part.

The portions of the receiving part that are between the closed ends of the blind holes 500a, 500b and the coaxial bore 53 of the receiving part are configured to be deformable portions 501a, 501b.

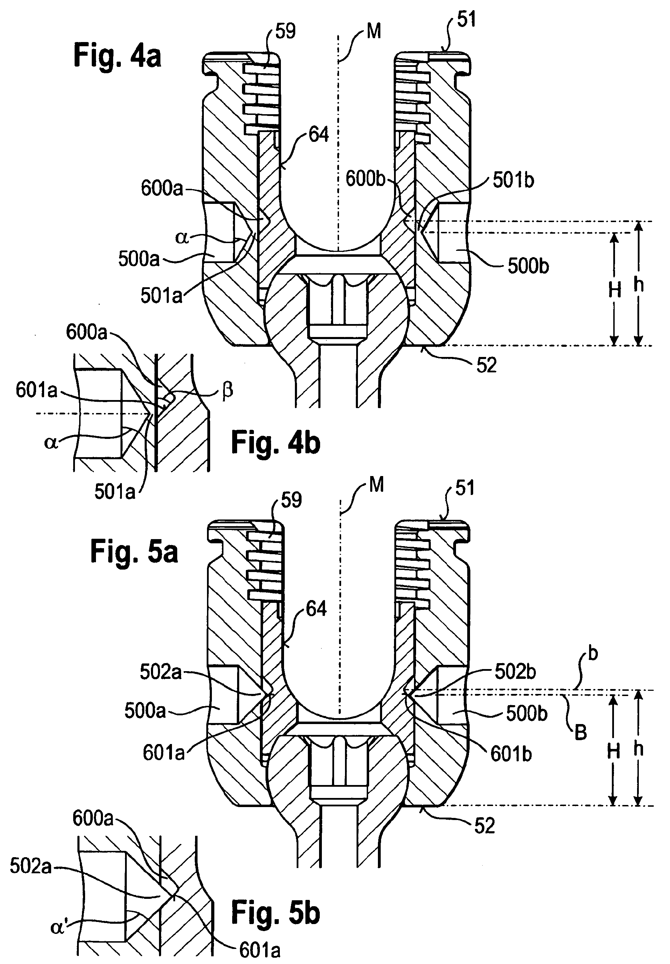

The pressure element 6 correspondingly includes two recesses 600a, 600b which are 180° offset from each other and 90° offset from the channel formed by the U-shaped recess 64. The recesses 600a, 600b have a center axis a, b, respectively, which is perpendicular to the bore axis M. In the embodiment shown, the recesses 600a, 600b have a conical shape. The downwardly extending flanks 601 a, 601b of the recesses 600a, 600b each include an angle β of approximately 45° with the central bore axis M. As shown in Fig. 4a and 4b, when the pressure element 6 is inserted such that it rests on the head 4 of the screw element, the central axis a, b of the recesses 600a, 600b has a distance h from the second end 52 of the receiving part 5 that is greater than the distance H of the central axis A, B of the blind holes 500a, 500b. In other words, the recesses 600a, 600b are arranged above the blind holes 500a, 500b.

The distance between the recesses and the blind holes is such that when the deformable portions 501a, 501b are deformed by applying a force via for example, a crimping tool to the blind holes 500a, 500b, the deformed material protrudes from the inner wall of the receiving part and presses onto the lower flanks 601a, 601b of the recesses 600a, 600b, respectively, to exert a downward force onto the pressure element 6. As shown in Figs. 5a and 5b, deforming the deformable portions 5a, 5b results in deformed portions 502a, 502b which exert pressure on the lower flank 601 a, 601 b of the recesses 600a, 600b of the pressure element 6. For example, after deformation, the angle α is approximately 45°, which is approximately the same as the angle of the lower flank 601 a, 601b. The blind holes 500a, 500b with their respective deformable portions 501a, 501b and the recesses 600a, 600b are constructed such that by deforming the deformable portions 501a, 501b into deformed portions 502a, 502b which engage the recesses 600a, 600b, the resulting force onto the pressure element 6 generates a preload onto the head 4, which clamps the head by means of friction. By selecting the sizes of blind holes and the recesses and their position, a desired friction force can be achieved. By this friction force the head can be maintained in a desired angular position and can be moved out of this position by applying a force greater than the friction force either to the screw element or to the receiving part. Simultaneously, the pressure element is secured against rotation and secured against escaping through the top end 51 of the receiving part. The recesses 600a, 600b provide space for accommodating a part of the deformed material. Also, the recesses 600a, 600b provide space for the protrusions 502a, 502b when the pressure element 6 moves downward to finally lock the head.

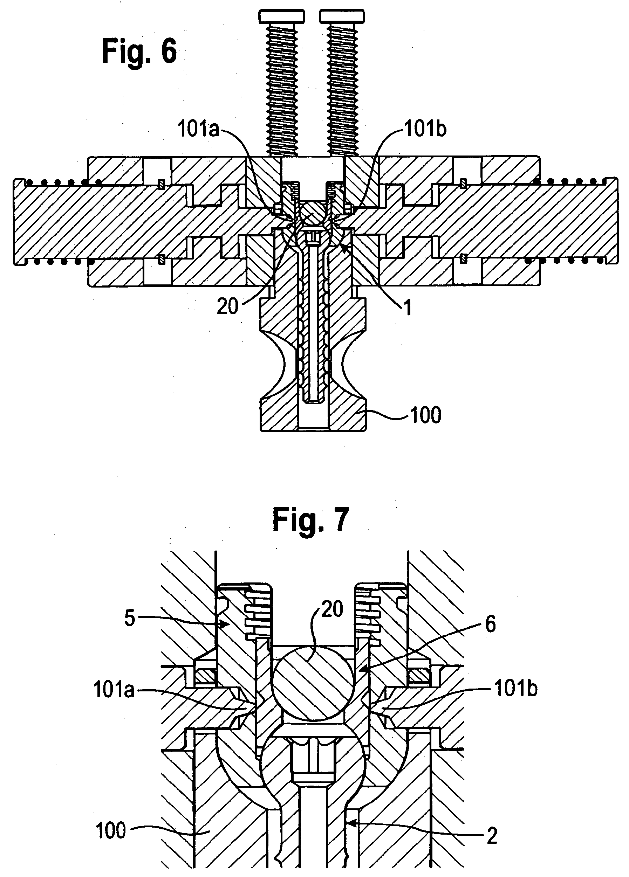

A method for manufacturing the polyaxial bone anchoring device is explained with reference to Figs. 6 and 7 and 4 to 5. A crimping tool shown in Figs. 6 and 7 generally comprises a holder 100 for the bone anchoring device, which serves for fixing the receiving part 5 with inserted screw element 2 and pressure element 6 as it is shown in Figs. 4a and 4b. The rod 20 may be inserted for providing a counter-force to avoid deformation of the free legs 65, 66 of the pressure element. The crimping tool further includes two crimping tips 101a, 101b, which are arranged 180° offset from each other and are dimensioned to be introduced into the blind holes 500a, 500b and to deform the deformable portions 501a, 501b, so that the displaced material, which forms the deformed portions 502a, 502b, engages the recesses 600a, 600b of the pressure element. As can be seen in particular in Fig. 7, the crimping tips 101a, 101b have an angle, which is more acute than that of the bottom of the blind hole 500a, 500b. The crimping tips 101a, 101b deform the deformable portion such that the deformed portion presses onto the lower flank 601 a, 601 b of the recess 600a, 600b, respectively. Thereafter, the crimping tips are retracted. The crimping process can be force-actuated and/or path-controlled.

After the crimping tips are retracted, the polyaxial anchoring device can be removed from the holder 100. The polyaxial bone anchoring device is then in a pre-assembled state with the screw element 2 being inserted and the pressure element being held in such a way that it exerts a slight preload onto the head which frictionally holds the head in an angular position.

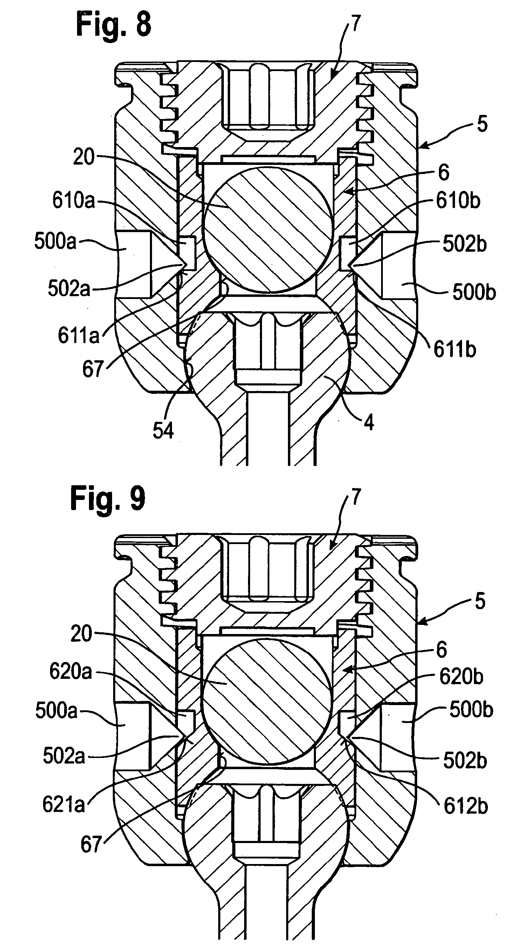

It shall be noted that the shape of the blind holes may vary. In particular, the angle of the conical bottom may vary or the bottom may have rounded or other shape. The recesses provided at the pressure element 6 may also have a different shape. As shown in Fig. 8, the recesses 610a, 610b can have, for example, a substantially rectangular cross-section. A lower side of the recess comprises an inclined edge 611a, 611b for engagement with the deformed portions 502a, 502b.

As shown in Fig. 9, the cross-section of the recesses 620a, 620b of the pressure element can be for example trapezoidal, with an inclined lower flank 621a, 621b for engagement with the deformed portions 502a, 502b.

All parts of the bone anchoring device are made of a body-compatible material, such as a body-compatible metal, for example, titanium, body-compatible metal alloys such as, for example, Nitinol or from a body-compatible plastic material, such as, for example, polyether ether ketone (PEEK) or combinations thereof.

Usually, several bone anchoring devices are necessary for stabilizing bone parts or vertebrae with the rod. In use, the bone anchoring devices are pre-assembled as shown in Fig. 5a, 5b. The screw members are screwed into the bone or a vertebra. Then, the receiving parts are pivoted by applying a force greater than the friction force until each receiving part has the correct orientation for the insertion of the rod. Due to the friction force, each receiving part is held in this angular position. Thereafter, the rod, which connects the bone anchoring devices, is inserted and the inner screw is tightened to move the pressure element downwards to lock the head in the seat so that the angular position of the screw member with respect to the receiving part is fixed. The rod is simultaneously fixed by the inner screw. Since the deformed portions 502a, 502b engage only the lower flank of the recesses provided at the pressure element, the recesses provide enough space for the deformed portions to allow a downward movement of the pressure element.

Further modifications of the previously described embodiment are conceivable. For example, only one deformed portion at the receiving part and one corresponding recess at the pressure element is sufficient. However, more than two deformed portions and corresponding recesses can also be provided.

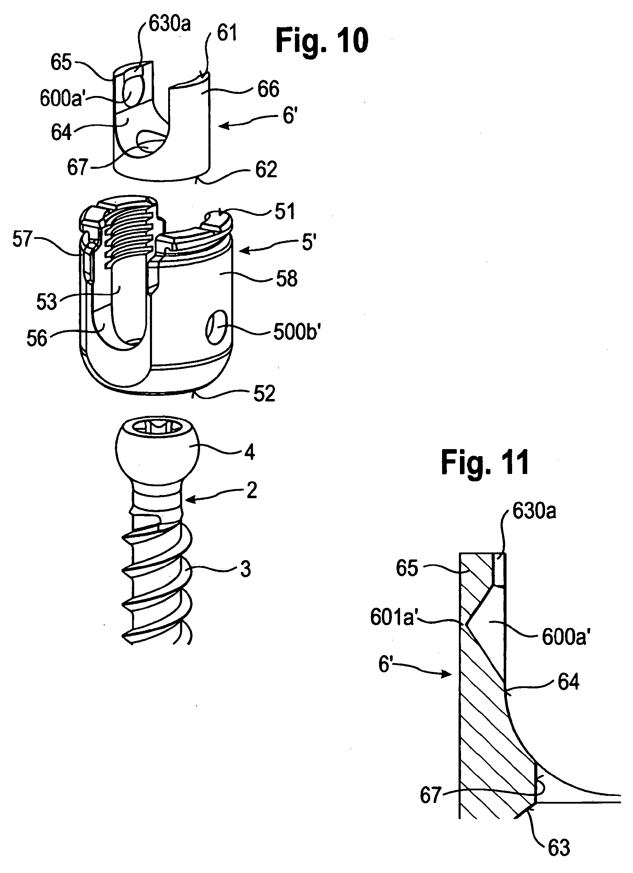

A second embodiment of the bone anchoring device is described with reference to Figs. 10 to 13. Parts or portions that are identical or similar to the previously decribed embodiment are designated with the same reference numerals and the decription thereof will not be repeated. The second embodiment differs from the first embodiment mainly in that the functions of the pressure element and the receiving part with respect to the provisional fixation with preload onto the head are reversed.

As can be seen in Fig. 9, the receiving part 5' has instead of the blind holes 500a, 500b two through holes 500a', 500b'. Although recesses at the inner wall of the receiving part instead of through holes would be sufficient, providing through holes is easier to manufacture and allows to upgrade existing receiving parts that have the blind holes of the first embodiment.

The pressure element 6' has two recesses 600a', 600b', arranged offset by 180°, that extend from the inner wall of the channel 64 into the legs 65, 66, respectively. The recesses can have a substantially triangular cross-section with a taper of approximately 22,5° similar to that of the blind holes 500a, 500b of the receiving part of the first embodiment. At the upper edge of the recesses a rectangular recess 630a, 630b is provided, respectively, the depth of which is smaller than that of the recesses 600a', 600b'. The recesses 630a, 630b are optional and may facilitate the insertion of a crimping tool.

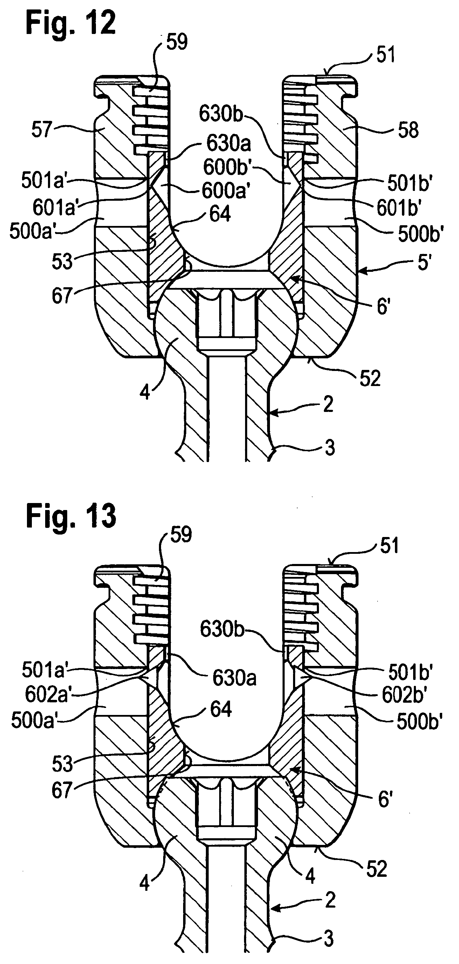

Between the outer surface of the pressure element 6' and the bottom of the recesses 600a', 600b', deformable portions 601a', 601b' are formed that can be deformed into deformed portions 602a', 602b' as shown in Fig. 13. In the pre-assembled and non-deformed state, as shown in Fig. 12, the pressure element 6' is situated in the receiving part 5' in such a position that it rests on the head 3 and the deformable portions are slightly below the upper wall portion of the through holes 500a', 500b'. Then, crimping tips (not shown) are introduced into the recesses 600a', 600b' and the deformable portions 601a', 601b' are deformed towards the outside. the deformed portions 602a', 602b' abut against the upper wall portion (501a', 501b') of the through holes 500a', 500b' at the inner side of the receiving part 5' as shown in Fig. 13. The deformed portions 602a', 602b' have then a taper of around 45°. When the deformed portions abut against the upper wall portion (501a', 501b') of the through holes, a downward force is exerted onto the head which clamps the head by friction.

The shape of the recesses and blind holes of the embodiments described is not limited to the tapered form. Also the angles of the taper are not limited to the values described. Other shapes are possible that achieve also a downwardly directed force when the deformable portions are deformed.

For the anchoring element, all kinds of anchoring elements can be used and combined with a receiving part. These anchoring elements are e.g. screws of different lengths, with different diameters, cannulated screws, screws with different thread forms, nails, hooks, etc. The head and the shaft can be separate parts which are connectable to each other.

The shape of the receiving part is not limited to the embodiment shown. For example, the receiving part can have an asymmetric end portion for allowing a greater pivot angle of the screw member to one side. Also, it is possible to have a recess allowing the rod to be introduced from the side instead of being introduced from the top or a closed recess through which the rod has to be guided. Various kinds of locking devices, including two- or more-part locking devices, outer nuts, outer caps, bayonet locking devices or others are possible.

In a further modification, the receiving part is configured to allow the introduction of the screw element from the bottom end.

## Claims

1. A polyaxial bone anchoring device including
an anchoring element (2) having a shaft (3) for anchoring in a bone and a head (4);
a receiving part (5, 5') having a top end (51) and a bottom end (52), a channel (56) for receiving a rod therein and a passage (53, 54, 55) extending from the top end (51) to the bottom end (52) and including a seat (54) for receiving the head (4);
a pressure element (6) which is arranged in the passage (53) and configured to exert pressure onto the head (4), wherein the pressure element is a solid member without a spring portion, and wherein the head is pivotable with respect to the receiving part (5) and can be locked at an angle by means of the pressure element (6);
the pressure element (6, 6') comprising at least one recess (600a, 600b; 610a, 610b; 620a, 620b) facing the inner wall of the receiving part or a deformable portion (601a', 601b') at its outer wall;
the receiving part (5) including at least one deformable portion (501a, 501b) provided at its inner wall, which upon deformation engages the recess (600a, 600b; 610a, 610b; 620a, 620b) at the pressure element or including a recess (500a', 500b') open at least to its inner wall;
wherein the deformable portion (502a, 502b; 601a', 601b') and the recess (600a, 600b; 610a, 610b; 620a, 620b; 500a', 500b') are arranged such that by engagement of the deformable portion with the recess a force is exerted by the pressure element (6) onto the head that maintains the head at an angular position by friction before locking the head, and
wherein the deformable portion (501a, 501 b, 601a', 601b') is configured to plastically deform into a deformed portion (502a, 502b, 602a', 602b') extending into the passage (53).

2. The polyaxial bone anchoring device of claim 1, wherein, when the pressure element (6) is inserted into the receiving part (5) and rests upon the head (4), the recess (600a, 600b) has a lower edge (601a, 601b, 611a, 611b; 621a, 621b) that is located above the deformable portion (501 a, 501b) in an axial direction towards the first end (51).

3. The polyaxial bone anchoring device of claim 2, wherein the distance in axial direction between the lower edge of the recess and the deformable portion (501a, 501b) is such that the deformed portion exerts a pressure onto the pressure element, when engaging the lower edge (601a, 601b; 611a, 611b; 621a, 621b) of the recess to generate a defined friction force between the head and the pressure element.

4. The polyaxial bone anchoring device of one of claims 1 to 3, wherein the receiving part includes a blind hole (500a, 500b) extending from the outer wall of the receiving part towards the inner wall and wherein the deformable portion (501a, 501b) is arranged between the inner wall and the blind hole.

5. The polyaxial bone anchoring device of one of claims 1 to 4, wherein the deformable portion (501a, 501b) is deformable into a tapered protrusion extending from the inner wall of the receiving part into the passage.

6. The polyaxial bone anchoring device of claim 4 or 5, wherein a closed end of the blind hole is tapered and includes an angle (β) with the central axis (M) of the passage of less than 45°, preferably of around 22,5°.

7. The polyaxial bone anchoring device according to one of claims 1 to 6, wherein the recess (600a, 600b; 610a, 610b; 620a, 620b) has an inclined lower edge (601a, 611a, 621a), which includes an angle with the central axis (M) of the passage of preferably around 45°.

8. The polyaxial bone anchoring device according to one of claims 1 to 7, wherein the recesses (600a, 600b) provide space for the deformed portion (502a, 502b) in an axial direction towards the first end (51).

9. The polyaxial bone anchoring device of claims 1 to 8, wherein at least two deformable portions (501a, 501b) and corresponding recesses (600a, 600b; 610a, 610b; 620a, 620b) arc provided, which are offset by 180° in a circumferential direction.

10. The polyaxial bone anchoring device of claim 1, wherein the deformable portion (601a', 601b') is provided at the pressure element (6') which upon deformation engages an upper edge (501a', 501b') of the recess (500a', 500b') provided at the receiving part (5').

11. The polyaxial bone anchoring device of one of claims 1 to 10, wherein the pressure element (6, 6')) includes a top end (61) and a bottom end (62), a coaxial bore (67), a spherical recess (63) at the bottom end and a cylindrical or U-shaped recess (64) at the top end and wherein the cylindrical or U-shaped recess (64) is aligned with the recess (56) of the receiving part.

12. A polyaxial bone anchoring device including
an anchoring element (2) having a shaft (3) for anchoring in a bone and a head (4);
a receiving part (5) having a top end (51) and a bottom end (52), a channel (56) for receiving a rod therein and a passage (53, 54, 55) extending from the top end (51) to the bottom end (52) and including a seat (54) for receiving the head (4);
a pressure element (6) which is arranged in the passage (53) and configured to exert pressure onto the head (4), wherein the pressure element is a solid member without a spring portion, wherein the head is pivotable with respect to the receiving part (5) and can be locked at an angle by means of the pressure element (6);
the pressure element (6) comprising at least one recess (600a, 600b; 610a, 610b; 620a, 620b) or a protrusion (602a', 602b') obtained by plastic deformation and facing the inner wall of the receiving part;
the receiving part comprising at least one protrusion (502a, 502b) obtained by plastic deformation or a recess (500a', 500b') provided at its inner wall, wherein, when the pressure element is inserted into the receiving part and rests on the head, the protrusion (502a, 502b, 602a', 602b') engages an edge (601a, 601b; 611a 611b; 621a, 621b, 501a', 501b') of the recess such that by the engagement a force is exerted by the pressure element onto the head (4) to maintain the head at an angular position by friction before locking the head.

13. The polyaxial bone anchoring device of claim 12, wherein at least two protrusions (502a, 502b, 602a', 602b') and corresponding recesses (600a, 600b; 610a, 610b; 620a, 620b, 500a', 500b')) are provided, which are offset by 180°.

## Patentansprüche

1. Polyaxiale Knochenverankerungsvorrichtung umfassend
ein Verankerungselement (2) mit einem Schaft (3) zum Verankern in einem Knochen und einem Kopf (4);
ein Aufnahmeteil (5, 5') mit einem oberen Ende (51) und einem unterem Ende (52), einem Kanal (56) zur Aufnahme eines Stabes darin und einen Durchlass (53, 54, 55), welcher sich von dem oberen Ende (51) zu dem unteren Ende (52) erstreckt und einen Sitz (54) zum Aufnehmen des Kopfes (4) umfasst;
ein Druckelement (6) welches in dem Durchlass (53) angeordnet ist und ausgebildet ist Druck auf den Kopf (4) auszuüben, wobei das Druckelement ein festes Teil ohne einen Federabschnitt ist, und wobei der Kopf in Bezug auf das Aufnahmeteil (5) schwenkbar ist und mittels des Druckelements (6) unter einem Winkel verriegelt werden kann;
wobei das Druckelement (6, 6') zumindest eine Ausnehmung (600a, 600b; 610a, 610b; 620a, 620b) aufweist, welche der Innenwand des Aufnahmeteils zugewandt ist, oder einen deformierbaren Abschnitt (601a', 601b') an seiner Außenwand;
wobei das Aufnahmeteil (5) zumindest einen deformierbaren Abschnitt (501a, 501b) umfasst, welcher an seiner Innenwand vorgesehen ist und bei Deformation mit der Ausnehmung (600a, 600b; 610a, 610b; 620a, 620b) in dem Druckelement in Eingriff gelangt, oder eine Ausnehmung (500a', 500b') umfasst, welche zumindest zu seiner Innenwand hin offen ist;
wobei der deformierbare Abschnitt (502a, 502b; 601a', 601b') und die Ausnehmung (600a, 600b; 610a, 610b; 620a, 620b; 500a', 500b') so angeordnet sind, dass durch in-Eingriff-Bringen des deformierbaren Abschnitts mit der Ausnehmung eine Kraft durch das Druckelement (6) auf den Kopf ausgeübt wird, welche den Kopf vor dem Verriegeln des Kopfes durch Reibung in einer Winkelstellung hält, und
wobei der deformierbare Abschnitt (501a, 501b, 601a', 601b') dazu ausgebildet ist sich plastisch in einen deformierten Abschnitt (502a, 502b; 602a', 602b') zu deformieren, welcher sich in den Durchlass (53) erstreckt.

2. Polyaxiale Knochenverankerungsvorrichtung nach Anspruch 1, wobei, wenn das Druckelement (6) in das Aufnahmeteil (5) eingesetzt ist und auf dem Kopf (4) aufliegt, die Ausnehmung (600a, 600b) eine untere Kante (601a, 601b, 611a, 611b; 621a, 621b) aufweist, welche sich in einer axialen Richtung in Richtung des ersten Endes (51) oberhalb des deformierbaren Abschnitts (501a, 501b) befindet.

3. Polyaxiale Knochenverankerungsvorrichtung nach Anspruch 2, wobei der Abstand in axialer Richtung zwischen der unteren Kante der Ausnehmung und des deformierbaren Abschnitts (501a, 501b) derart ist, dass der deformierte Abschnitt einen Druck auf das Druckelement ausübt, wenn er mit der unteren Kante (601a, 601b; 611a, 611b; 621a, 621b) der Ausnehmung in Eingriff gelangt um eine definierte Reibungskraft zwischen dem Kopf und dem Druckelement zu erzeugen.

4. Polyaxiale Knochenverankerungsvorrichtung nach einem der Ansprüche 1 bis 3, wobei das Aufnahmeteil ein Sackloch (500a, 500b) umfasst, welches sich von der Außenwand des Aufnahmeteils in Richtung der Innenwand erstreckt, und wobei der deformierbare Abschnitt (501a, 501b) zwischen der Innenwand und dem Sackloch angeordnet ist.

5. Polyaxiale Knochenverankerungsvorrichtung nach einem der Ansprüche 1 bis 4, wobei der deformierbare Abschnitt (501a, 501b) in einen zulaufenden Vorsprung deformierbar ist, welcher sich von der Innenwand des Aufnahmeteils in den Durchlass erstreckt.

6. Polyaxiale Knochenverankerungsvorrichtung nach Anspruch 4 oder 5, wobei ein geschlossenes Ende des Sacklochs zulaufend ist und einen Winkel (β) mit der Zentralachse (M) des Durchlasses bildet, welcher kleiner als 45° ist, vorzugsweise etwa 22,5°.

7. Polyaxiale Knochenverankerungsvorrichtung nach einem der Ansprüche 1 bis 6, wobei die Ausnehmung (600a, 600b; 610a, 610b; 620a, 620b) eine schräge untere Kante (601a, 611a, 621a) aufweist, welche einen Winkel mit der Zentralachse (M) des Durchlasses von vorzugsweise etwa 45° bildet.

8. Polyaxiale Knochenverankerungsvorrichtung nach einem der Ansprüche 1 bis 7, wobei die Ausnehmungen (600a, 600b) für den deformierten Abschnitt (502a, 502b) in eine axiale Richtung in Richtung des ersten Endes (51) Platz bieten.

9. Polyaxiale Knochenverankerungsvorrichtung nach einem der Ansprüche 1 bis 8, wobei zumindest zwei deformierbare Abschnitte (501a, 501b) und zugehörige Ausnehmungen (600a, 600b; 610a, 610b; 620a, 620b) vorgesehen sind, welche um 180° in einer Umfangsrichtung versetzt sind.

10. Polyaxiale Knochenverankerungsvorrichtung nach Anspruch 1, wobei der deformierbare Abschnitt (601a', 601b') an dem Druckelement (6') vorgesehen ist, welcher bei Deformierung mit einer oberen Kante (501a', 501b') der Ausnehmung (500a', 500b'), welche an dem Aufnahmeteil (5') vorgesehen ist, in Eingriff gelangt.

11. Polyaxiale Knochenverankerungsvorrichtung nach einem der Ansprüche 1 bis 10, wobei das Druckelement (6, 6') ein oberes Ende (61) und ein unteres Ende (62) umfasst, eine koaxiale Bohrung (67), eine sphärische Ausnehmung (63) an dem unteren Ende (62) und eine zylindrische oder U-förmige Ausnehmung (64) an dem oberen Ende und wobei die zylindrische oder U-förmige Ausnehmung (64) mit der Ausnehmung (56) des Aufnahmeteils ausgerichtet ist.

12. Polyaxiale Knochenverankerungsvorrichtung umfassend
ein Verankerungselement (2) mit einem Schaft (3) zum Verankern in einem Knochen und einen Kopf (4);
ein Aufnahmeteil (5) mit einem oberen Ende (51) und einem unterem Ende (52), einem Kanal (56) zur Aufnahme eines Stabes darin und einen Durchlass (53, 54, 55), welcher sich von dem oberen Ende (51) zu dem unteren Ende (52) erstreckt und einen Sitz (54) zum Aufnehmen des Kopfes (4) umfasst;
ein Druckelement (6) welches in dem Durchlass (53) angeordnet ist und ausgebildet ist Druck auf den Kopf (4) auszuüben, wobei das Druckelement ein festes Teil ohne einen Federabschnitt ist, und wobei der Kopf in Bezug auf das Aufnahmeteil (5) schwenkbar ist und mittels des Druckelements (6) unter einem Winkel verriegelt werden kann;
wobei das Druckelement (6) zumindest eine Ausnehmung (600a, 600b; 610a, 610b; 620a, 620b) aufweist oder einen Vorsprung (602a', 602b'), welcher durch plastische Deformation erhalten ist und welcher der Innenwand des Aufnahmeteils zugewandt ist;
wobei das Aufnahmeteil zumindest einen Vorsprung (502a, 502b), welcher durch plastische Deformation erhalten ist, oder eine Ausnehmung (500a', 500b') an seiner Innenwand aufweist, wobei, wenn das Druckelement in das Aufnahmeteil eingesetzt ist und auf dem Kopf aufliegt, der Vorsprung (502a, 502b, 602a', 602b') mit einer Kante (601a, 601b; 611a, 611b; 621a, 621b, 501a', 501b') der Ausnehmung in Eingriff gelangt, so dass durch das in-Eingriff-Bringen eine Kraft durch das Druckelement auf den Kopf (4) ausgeübt wird um den Kopf vor dem Verriegeln des Kopfes durch Reibung in einer Winkelstellung zu halten.

13. Polyaxiale Knochenverankerungsvorrichtung nach Anspruch 12, wobei zumindest zwei Vorsprünge (502a, 502b, 602a', 602b') und zugehörige Ausnehmungen (600a, 600b; 610a, 610b; 620a, 620b, 500a', 500b') vorgesehen sind, welche um 180° versetzt sind.

## Revendications

1. Dispositif d'ancrage d'os polyaxial comprenant
un élément (2) d'ancrage pourvu d'une tige (3) destinée à être ancrée dans un os et d'une tête (4) ;
une partie (5, 5') de réception présentant une extrémité (51) supérieure et une extrémité (52) inférieure, un canal (56) destiné à recevoir une barre à l'intérieur de ce dernier et un passage (53, 54, 55) qui s'étend de l'extrémité (51) supérieure à l'extrémité (52) inférieure et comprend un siège (54) destiné à recevoir la tête (4) ;
un élément (6) de pression qui est agencé dans le passage (53) et configuré pour exercer une pression sur la tête (4), où l'élément de pression est un organe plein sans partie ressort, et où la tête peut pivoter par rapport à la partie (5) de réception et peut être verrouillée en position inclinée au moyen de l'élément (6) de pression ;
l'élément (6, 6') de pression comportant au moins un renfoncement (600a, 600b ; 610a, 610b ; 620a, 620b) orienté vers la paroi interne de la partie de réception ou une partie (601a', 601b') déformable au niveau de sa paroi externe ;
la partie (5) de réception comprenant au moins une partie (501a, 501b) déformable fournie au niveau de sa paroi interne, qui, au moment de sa déformation, vient en contact avec le renfoncement (600a, 600b ; 610a, 610b ; 620a, 620b) au niveau de l'élément de pression ou comprenant un renfoncement (500a', 500b') ouvert au moins vers sa paroi interne ;
où la partie (502a, 502b ; 601a', 601b') déformable et le renfoncement (600a, 600b ; 610a, 610b ; 620a, 620b ; 500a', 500b') sont agencés de telle sorte que lorsque la partie déformable vient en contact avec le renfoncement une force est exercée par l'élément (6) de pression sur la tête qui maintient la tête à une position angulaire par frottement avant de verrouiller la tête, et
où la partie (501a, 501b, 601a', 601b') déformable est configurée pour se déformer plastiquement en une partie (502a, 502b, 602a', 602b') déformée qui s'étend dans le passage (53).

2. Dispositif d'ancrage d'os polyaxial selon la revendication 1, où, lorsque l'élément (6) de pression est inséré dans la partie (5) de réception et repose sur la tête (4), le renfoncement (600a, 600b) présente un bord (601a, 601b, 611a, 611b ; 621a, 621b) inférieur qui est situé au-dessus de la partie (501a, 501b) déformable dans une direction axiale allant vers la première extrémité (51).

3. Dispositif d'ancrage d'os polyaxial selon la revendication 2, où la distance dans la direction axiale entre le bord inférieur du renfoncement et la partie (501a, 501b) déformable est telle que la partie déformée exerce une pression sur l'élément de pression, lorsqu'elle vient en contact avec le bord (601a, 601b ; 611a, 611b ; 621a, 621b) inférieur du renfoncement pour produire une force de frottement définie entre la tête et l'élément de pression.

4. Dispositif d'ancrage d'os polyaxial selon l'une quelconque des revendications 1 à 3, où la partie de réception comprend un trou (500a, 500b) borgne qui s'étend de la paroi externe de la partie de réception vers la paroi interne et où la partie (501a, 501b) déformable est agencée entre la paroi interne et le trou borgne.

5. Dispositif d'ancrage d'os polyaxial selon l'une quelconque des revendications 1 à 4, où la partie (501a, 501b) déformable peut être déformée en une protubérance conique qui s'étend de la paroi interne de la partie de réception dans le passage.

6. Dispositif d'ancrage d'os polyaxial selon la revendication 4 ou 5, où une extrémité fermée du trou borgne est conique et comprend un angle (β) avec l'axe (M) central du passage inférieur à 45°, se situant de préférence autour de 22,5°.

7. Dispositif d'ancrage d'os polyaxial selon l'une des revendications 1 à 6, où le renfoncement (600a, 600b ; 610a, 610b ; 620a, 620b) présente un bord (601a, 611a, 621a) inférieur incliné, qui comprend un angle avec l'axe (M) central du passage se situant de préférence autour de 45°.

8. Dispositif d'ancrage d'os polyaxial selon l'une quelconque des revendications 1 à 7, où les renfoncements (600a, 600b) fournissent de l'espace pour la partie (502a, 502b) déformée dans une direction axiale vers la première extrémité (51).

9. Dispositif d'ancrage d'os polyaxial selon les revendications 1 à 8, où au moins deux parties (501a, 501b) déformables et renfoncements (600a, 600b ; 610a, 610b ; 620a, 620b) correspondants sont fournis, qui sont décalés de 180° dans une direction circonférentielle.

10. Dispositif d'ancrage d'os polyaxial selon la revendication 1, où la partie (601a', 601b') déformable est fournie au niveau de l'élément (6') de pression qui, lorsqu'elle se déforme, vient en contact avec un bord (501a', 501b') supérieur du renfoncement (500a', 500b'), fourni au niveau de la partie (5') de réception.

11. Dispositif d'ancrage d'os polyaxial selon l'une quelconque des revendications 1 à 10, où l'élément (6, 6') de pression comprend une extrémité (61) supérieure et une extrémité (62) inférieure, un alésage (67) coaxial, un renfoncement (63) sphérique au niveau de l'extrémité inférieure et un renfoncement (64) cylindrique ou en forme de U au niveau de l'extrémité supérieure et où le renfoncement (64) cylindrique ou en forme de U est aligné avec le renfoncement (56) de la partie de réception.

12. Dispositif d'ancrage d'os polyaxial comprenant
un élément (2) d'ancrage pourvu d'une tige (3) destinée à être ancrée dans un os et d'une tête (4) ;
une partie (5) de réception présentant une extrémité (51) supérieure et une extrémité (52) inférieure, un canal (56) destiné à recevoir une barre à l'intérieur de ce dernier et un passage (53, 54, 55) qui s'étend de l'extrémité (51) supérieure à l'extrémité (52) inférieure et comprend un siège (54) destiné à recevoir la tête (4) ;
un élément (6) de pression qui est agencé dans le passage (53) et configuré pour exercer une pression sur la tête (4), où l'élément de pression est un organe plein sans partie ressort, où la tête peut pivoter par rapport à la partie (5) de réception et peut être verrouillée au niveau d'un angle au moyen de l'élément (6) de pression ;
l'élément (6) de pression comportant au moins un renfoncement (600a, 600b ; 610a, 610b ; 620a, 620b) ou une protubérance (602a', 602b') obtenue par déformation plastique et orientée vers la paroi interne de la partie de réception ;
la partie de réception comportant au moins une protubérance (502a, 502b) obtenue par déformation plastique ou un renfoncement (500a', 500b') fourni au niveau de sa paroi interne, où, lorsque l'élément de pression est inséré dans la partie de réception et repose sur la tête, la protubérance (502a, 502b, 602a', 602b') vient en contact avec un bord (601a, 601b; 611a, 611b ; 621a, 621b, 501a', 501b') du renfoncement de telle sorte que par le contact une force est exercée par l'élément de pression sur la tête (4) pour maintenir la tête à une position angulaire par frottement avant de verrouiller la tête.

13. Dispositif d'ancrage d'os polyaxial selon la revendication 12, où au moins deux protubérances (502a, 502b, 602a', 602b') et des renfoncements (600a, 600b ; 610a, 610b ; 620a, 620b, 500a', 500b') correspondants sont fournis, qui sont décalés de 180°.
